# EUROPEAN PATENT SPECIFICATION

(11) **EP 3 458 017 B1**
(45) Date of publication and mention of the grant of the patent: **17.03.2021**
(21) Application number: 17735291.1
(22) Date of filing: 21.06.2017
(51) Int. Cl.: A61K 8/27, A61K 8/35, A61Q 11/00, A61K 8/19

(54) **ORAL CARE COMPOSITIONS**
MUNDPFLEGEMITTEL
COMPOSITIONS DE SOINS ORALES

(30) Priority: 24.06.2016 US 201662354285 P
(43) Date of publication of application: 27.03.2019
(73) Proprietor: Colgate-Palmolive Company, New York, NY 10022 (US)
(72) Inventor: CHEN, Dandan, Bridgewater New Jersey 08807 (US); YANG, Ying, Monmouth Junction New Jersey 08852 (US); D'AMBROGIO, Robert, Princeton New Jersey 08540 (US); THOMSON, Paul, Piscataway New Jersey 08854 (US); TRIVEDI, Harsh Mahendra, Hillsborough New Jersey 08844 (US); PRENCIPE, Michael, West Windsor New Jersey 08550 (US); MASTERS, James Gerard, Ringoes NJ 08551 (US); JARACZ, Stanislav, Somerset New Jersey 08873 (US)
(74) Representative: Wibbelmann, Jobst
(86) International application number: PCT/US2017/038487
(87) International publication number: WO 2017/223169

(56) References cited:
- WO-A1-2015/095627
- WO-A2-2010/138492
- US-A1- 2010 027 899

## Description

This invention relates to oral care compositions comprising a phenolic alkanone, e.g., zingerone, a stannous ion source, zinc oxide, and zinc citrate.

### BACKGROUND

Stannous fluoride is known as useful fluoride source for dentifrices. Stannous fluoride possesses an advantage over some other ionic fluorides, including sodium fluoride, in that it is antimicrobial - it kills bacteria in the mouth by interfering with the bacterial metabolic processes.

Zinc is also a known antimicrobial agent used in toothpaste compositions and a known essential mineral for human health, and has been reported to help strengthen dental enamel and to promote cell repair.

Certain patient populations suffer from conditions that produce inflammation in the oral cavity, such as gum disease and gingivitis. There is thus a need for improved toothpaste formulations that have increased anti-inflammatory properties, and that provide additional antimicrobial benefits.

### BRIEF SUMMARY

It has been surprisingly found that the inclusion of zingerone in an oral care composition comprising a source of stannous ions, zinc oxide and zinc citrate, selected at certain concentrations and amounts, unexpectedly increases the anti-inflammation efficacy of the oral care formulas in the oral cavity of a user.

In one aspect the present disclosure provides an oral care composition (Composition 1.0) comprising:
a. a phenolic alkenone comprising zingerone, where the zingerone is present in an amount from 0.01 to 1 wt%, based on the total weight of the composition,
a. zinc oxide;
b. zinc citrate; and
c. a stannous ion source, wherein the source of stannous ions is stannous fluoride in an amount of 0.1 to 2 wt %, based on the total weight of the composition;
wherein the zinc citrate is present in an amount of from 0.25 to 1.0 wt % and zinc oxide is present in an amount of from 0.75 to 1.25 wt %, based on the total weight of the composition.

In another embodiment, the invention encompasses the oral compositions as defined in the claims for use in a method to improve oral health comprising applying an effective amount of said oral composition to the oral cavity of a subject in need thereof in a method to
i. reduce or inhibit formation of dental caries,
ii. and/or
iii.clean the teeth and oral cavity.

### DETAILED DESCRIPTION

As used herein, the term "oral composition" means the total composition that is delivered to the oral surfaces. The composition is further defined as a product which, during the normal course of usage, is not, the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

### Phenolic Alkanone, e.g. Zingerone

Zingerone (4-(3-methoxy-4-hydroxyphenyl)-butan-2-one, also known as vanillylacetone), has the formula:

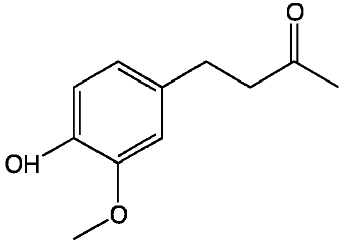

Zingerone is a key component of the pungency of ginger. Zingerone is a member of both the methoxyphenol family and its related derivatives, which have a basic phenolic ring with a methoxy group attached to benzene ring, and the phenolic alkanone group, which is characterized by having an alkanone group attached to the phenolic ring. Zingerone has varied pharmacological properties that include antioxidant, anti-inflammatory, anticancer, lipolytic, antiemetic, antidiarrhoeal, immuno-stimulatory and antimicrobial activities. See Ahman, B., et al, "A Review on Pharmacological Properties of Zingerone (4-(4-Hydroxy-3-methoxyphenyl)-2-butanone)", ScientificWorldJournal.2015; 2015: 816364. Zingerone is created by the heating or cooking of fresh ginger, which transforms gingerol to zingerone by a retroaldol reaction. Zingerone is present in ginger an amount of about 9.25%, and can be synthesized by, inter alia, the process disclosed in U.S. Patent No. 2,381,210 to Cotton.

In some embodiments, the phenolic alkanone also contains a methoxy group on the phenyl ring - i.e., it is a methoxyphenolic alkanone. Generally, the phenolic alkanone is present in an amount of from 0.01% to 1% (e.g., 0.05% to 0.5%; e.g., 0.05% to 0.35%; e.g., 0.1%, 0.2%, or 0.3%) by weight of the composition. In some preferred embodiments, the phenolic alkanone is a methoxyphenolic alkanone, e.g., zingerone.

### Zinc salts

The compositions of the present disclosure contain zinc oxide and zinc citrate, preferably in a ratio of zinc oxide (wt.%) to zinc citrate (wt%) of from 1.5:1 to 4.5:1, e.g., 2:1, 2.5:1, 3:1, 3.5:1, or 4:1. Although the zinc citrate can be used in the present compositions in any hydrated or anhydrous form, the wt. percentages used herein refer to the trihydrate; i.e., zinc citrate trihydrate. In some embodiments, the zinc citrate is present in an amount of from 0.25 to 1.0 wt% (e.g., 0.5 wt. %) and zinc oxide is present in an amount of from 0.75 to 1.25 wt% (e.g., 1.0 wt. %) based on the weight of the oral care composition.

### Stannous Ion Source

The oral care compositions may further include one or more stannous ion sources e.g. stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof. In some preferred embodiments, the fluoride source is stannous fluoride. In some embodiments, the stannous fluoride is present in an amount of 0.1 wt. % to 2 wt. %, e.g. 0.1 wt% - 0.6 wt.%, e.g., 0.4-0.5 wt.% of the total composition weight.

### Abrasives

The compositions of the disclosure can include abrasives. Examples of suitable abrasives include silica abrasives, such as standard cleaning silicas, high cleaning silicas or any other suitable abrasive silicas. Additional examples of abrasives that can be used in addition to or in place of the silica abrasives include abrasives such as sodium metaphosphate, potassium metaphosphate, aluminum silicate, calcined alumina, bentonite or other siliceous materials, or combinations thereof.

Silica abrasive polishing materials useful herein, as well as the other abrasives, generally have an average particle size ranging between 0.1 and 30 microns, such as between 5 and 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the disclosure include silica gels and precipitated amorphous silica having an oil absorption value of less than 100 cc/100 g silica, such as from 45 cc/100 g to 70 cc/100 g silica. Oil absorption values are measured using the ASTA Rub-Out Method D281. In certain embodiments, the silicas are colloidal particles having an average particle size of from 3 microns to 12 microns, and from 5 to 10 microns. Examples of low oil absorption silica abrasives useful in the practice of the disclosure are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging from 7 to 10 microns in diameter, and an oil absorption of less than 70 cc/100 g of silica is an example of a low oil absorption silica abrasive useful in the practice of the present disclosure.

Any suitable amount of silica abrasive can be employed. In some embodiments, the present compositions include a synthetic amorphous precipitated abrasive silica in an amount of, e.g., 1% - 25% by wt., e.g., 8% - 25% by wt., e.g., 10% - 15% by wt. In some embodiment, the present compositions further include a high cleaning silica, in an amount of, e.g., 1% - 15% by wt., e.g., 5% - 10%, e.g., 7% by wt.

### Alkali Phosphate Salts

In some embodiments, the present compositions can include an effective amount of one or more alkali phosphate salts, e.g., sodium, or potassium salts, e.g., selected from alkali dibasic phosphate and alkali pyrophosphate salts. Suitable alkali phosphate salts include those selected from sodium phosphate dibasic, potassium phosphate dibasic, tetrasodium pyrophosphate, tetrapotassium pyrophosphate, sodium tripolyphosphate, disodium hydrogenorthophoshpate, monosodium phosphate, pentapotassium triphosphate and mixtures of any of two or more of these, e.g., in an amount of 1-20%, e.g., 2-8%, e.g., 2-5%, by weight of the composition. In some embodiments, the polyphosphate is sodium tripolyphosphate, in an amount of from 1 - 5 wt %, e.g., about 3 wt%.

### Polymers

The oral care compositions of the present disclosure also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include PVM/MA copolymers. The term "PVM/MA copolymer" as used herein is intended to include copolymers of maleic anhydride or acid with another polymerizable ethylenically unsaturated monomer, preferably methyl vinyl ether/maleic anhydride. In some embodiments, the copolymers include 1:4 to 4:1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139 (M.W. 500,000), AN 119 (M.W. 250,000) and S-97 Pharmaceutical Grade (M.W. 70,000), of GAF Chemicals Corporation. In some embodiments, the present compositions include a PVM/MA copolymer, in an amount of from 0.1-5%, e.g., 0.2-2%, e.g., 0.3-1%, e.g., Gantrez S-97.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2-methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

### Thickeners

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as xanthan gum, karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used. In some embodiments, the present compositions include carboxymethyl cellulose in an amount of from 0.1 wt.% - 1.5 wt.%.

### Surfactants

The present compositions can include one or more surfactants, for example anionic surfactants and amphoteric (zwitterionic) surfactants. Examples of suitable anionic surfactants include, for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOS0₃X, wherein m is 6-16, e.g., 10, n is 1-6, e.g., 2, 3 or 4, and X is Na or , for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃o alkyl. In particular embodiments, the anionic surfactant is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %-2%, and optimal concentrations depend on the particular formulation and the particular surfactant. In one embodiment, the anionic surfactant is present at from 0.03% to 5% by weight, e.g., 1%-1%, e.g. 1.5%-2% by weight.

In some embodiments, the compositions of the present disclosure include an amphoteric surfactant. Suitable amphoteric surfactants include betaines and sultaines. In some embodiments, the amphoteric surfactant comprises a betaine having a quaternary ammonium or phosphonium ion as the cationic group and a carboxylate group as the anionic group; for example a betaine having a quaternary ammonium ion as the cationic group and a carboxylate group as the anionic group (i.e., a quaternary ammonium carboxylate betaine). Typical alkyldimethyl betaines include, but are not limited to, decyl dimethyl betaine or 2-(N-decyl-N, N-dimethylammonia)acetate, coco dimethyl betaine or 2-(N-coco N, N-dimethylammonia)acetate, myristyl dimethyl betaine, palmityl dimethyl betaine, lauryl dimethyl betaine, cetyl dimethyl betaine, stearyl dimethyl betaine, etc. The amidobetaines similarly include, but are not limited to, cocoamidoethylbetaine, cocoamidopropyl betaine and the like. In one embodiment, the betaine is cocamidopropyl betaine. Two examples of betaine surfactants that can be used are EMPIGEN™ BS/CA from Huntsman, and Tegobetaine F50 from BASF. Other suitable amphoteric surfactants include amine oxides.

In some embodiments, the compositions of the present disclosure comprise a single amphoteric surfactant. In some embodiments, the amphoteric surfactant is present in an amount of about 0.5 wt % to about 5 wt %, e.g. about 0.5 wt % to about 1.5 wt %, e.g. about 1 wt %.

In some embodiments, the surfactant system comprises a amphoteric surfactant and an anionic surfactant in a weight ratio of about 1:1 to about 1:3. In some embodiments, the ratio of amphoteric:anionic surfactant is about 1:1.5 to about 1:2, for example about 1:1.75.

Illustrative nonionic surfactants that can be used in the present compositions can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the present disclosure comprise a nonionic surfactant selected from poloxamers (e.g., poloxamer 407), polysorbates (e.g., polysorbate 20), polyoxyl hydrogenated castor oils (e.g., polyoxyl 40 hydrogenated castor oil), and mixtures thereof. When present, the nonionic surfactant can be present in an amount of from 0.1% to 3%, for example 0.1% to 1.5% by weight of the total composition.

### Humectants

Within certain embodiments of the oral compositions, it is also desirable to incorporate one or more humectants to reduce evaporation and also contribute towards preservation by lowering water activity. Certain humectants can also impart desirable sweetness or flavor to the compositions. The humectant(s), on a pure humectant basis, are generally present in an amount of from 15% to 70% by weight, for example 30% to 65% by weight, for example 45-55% by weight of the composition.

Suitable humectants include edible polyhydric alcohols such as glycerine, sorbitol, xylitol, propylene glycol, polyethylene glycol, as well as other polyols and mixtures of these humectants. Mixtures of glycerine, propylene glycol and polyethylene glycol may be used in certain embodiments as the humectant component of the compositions herein. For example, in some embodiments, one or both of polyethylene glycol and propylene glycol is included, each in an amount of 1%-6% by weight.

### Flavoring Agents

The present oral care compositions may also include a flavoring agent. Flavoring agents which are used in the present compositions include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

### Chelating and anti-calculus agents

The present oral care compositions also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present compositions are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.5 wt. % pyrophosphate ions, 0.9 - 3 wt. %. The pyrophosphates also contribute to preservation of the compositions by lowering water activity.

### Buffer

The present compositions can further include one or more buffering systems. One suitable buffering system is a mixture of citric acid and citrate ion, for example in a ratio of citric acid to citrate ion of from 1:3 to 1:10, e.g., 1:3 to 1:7, e.g. from 1:4 to 1:6, e.g. about 1:5, by weight, based on the weight of anhydrous citric acid and trisodium citrate dihydrate. Thus, in some embodiments, the present compositions include anhydrous citric acid in an amount of from 0.1-3 wt. %, e.g., 0.1-1 wt. %, e.g., 0.4-0.8 wt. %, e.g., about 0.6 wt. %; and trisodium citrate dihydrate, in an amount of from 0.1-5 wt. %, e.g., 2-4 wt. %, e.g., about 3 wt. %.

### Water

Water is present in the oral compositions of the invention. Water, employed in the preparation of commercial oral compositions should be deionized and free of organic impurities. Water commonly makes up the balance of the compositions and includes from 5% - 40%, e.g., 5% - 25%, e.g., 5%-15%, e.g., about 8-10% by weight of the oral compositions. This amount of water includes the free water which is added, and does not include that amount which is introduced with other materials such as with sorbitol or silica or any components of the invention. The Karl Fischer method is a one measure of calculating free water.

### Basic Amino Acids

The basic amino acids which can be used in the compositions of the invention include not only naturally occurring basic amino acids, such as arginine, lysine, and histidine, but also any basic amino acids having a carboxyl group and an amino group in the molecule, which are water-soluble and provide an aqueous solution with a pH of 7 or greater.

Accordingly, basic amino acids include, but are not limited to, arginine, lysine, serine, citrullene, ornithine, creatine, histidine, diaminobutanoic acid, diaminoproprionic acid, salts thereof or combinations thereof. In a particular embodiment, the basic amino acids are selected from arginine, citrullene, and ornithine.

In certain embodiments, the basic amino acid is arginine, for example, L-arginine, or a salt thereof.

The compositions of the invention are intended for topical use in the mouth and so salts for use in the present invention should be safe for such use, in the amounts and concentrations provided. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

The oral care compositions of the present invention are for use in methods as defined in the claims, wherein the methods include applying to the oral cavity a safe and effective amount of the compositions described herein.

The present compositions (e.g., Composition 1.0 *et seq*) can be incorporated into oral compositions for the care of the mouth and teeth such as toothpastes, transparent pastes, gels, mouth rinses, sprays and chewing gum.

As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof

### Example 1

### Dentifrice Formulations

The following dentifrice formulations were prepared:
Formula A: Stannous fluoride, zinc oxide, zinc citrate
Formula B: Stannous fluoride, zinc oxide, zinc citrate, 0.1% Zingerone
Formula C: Stannous fluoride, zinc oxide, zinc citrate, 0.3% Zingerone

The compositions are shown in Table 1 below:

**Table 1**

| Description | Formula A (wt%) | Formula B (wt%) | Formula C (wt%) |
|---|---|---|---|
| DEMINERALIZED WATER | 8.8 | 8.8 | 8.8 |
| SODIUM SACCAHRIN | 0.80 | 0.80 | 0.80 |
| TRISODIUM CITRATE DIHYDRATE | 3.0 | 3.0 | 3.0 |
| CITRIC ACID ANHYDROUS | 0.6 | 0.6 | 0.6 |
| STANNOUS FLUORIDE | 0.454 | 0.454 | 0.454 |
| ZINC OXIDE | 1.0 | 1.0 | 1.0 |
| 99.0-101.0% GLYCERIN | 40.9 | 40.9 | 40.9 |
| POLYETHYLENE GLYCOL | 3.0 | 3.0 | 3.0 |
| PROPYLENE GLYCOL | 4.0 | 4.0 | 4.0 |
| THICKENERS (includes e.g., xanthan gum, carboxymentyl cellulose, microcrystalline cellulose/NaCMC) | 1.4 | 1.4 | 1.4 |
| PVP | 1.25 | 1.25 | 1.25 |
| DYE | 0.002 | 0.002 | 0.002 |
| ABRASIVES (includes, e.g., syn. amorph. ppt. silica, high cleaning silica, silicon dioxide) | 24.0 | 24.0 | 24.0 |
| SODIUM LAURYL SULFATE POWDER | 1.75 | 1.75 | 1.75 |
| COCAMIDOPROPYL BETAINE (30% SOL'N) | 1.0 | 1.0 | 1.0 |
| GANTREZ S-97 (16.5% SOL'N) | 0.606 | 0.606 | 0.606 |
| TITANIUM DIOXIDE COATED MICA | 0.15 | 0.15 | 0.15 |
| 85% SYRUPY PHOSPHORIC ACID FOOD GRADE | 0.60 | 0.60 | 0.60 |
| SODIUM TRIPHOSPHATE, TRIBASIC 12-HYDRATE | 1.00 | 1.00 | 1.00 |
| ZINC CITRATE TRIHYDRATE | 0.50 | 0.50 | 0.50 |
| SODIUM TRIPOLYPHOSPHATE - FCC GRADE | 3.0 | 3.0 | 3.0 |
| FLAVOR | 2.2 | 2.1 | 1.9 |
| ZINGERONE | --- | 0.10 | 0.30 |

### Example 2

### Determination of anti-inflammation efficacy of toothpaste formulas

Cytokine PGE2 was used as an inflammation marker to evaluate the anti-inflammation efficacy of toothpaste formulas. Treatments (2 minute each) were performed on human gingival tissue (Mattek Corporation, Ashland, MA) in the presence of IL-1b in the culture medium.

Each treatment was conducted on three tissues. The three trials were repeated showing consistent results. The percent reduction of PGE2 16 hours after the 2 minute toothpaste slurry treatments is shown in Table 2 below. Table 2 shows the results of Formulas A, B and C tested along with a comparative formulation containing 0.3% triclosan. Each of the data are reported as the average of three trials of experiments.

**Table 2**

| % Reduction of PGE2 | |
|---|---|
| Formula | % Reduction of PGE2 |
| A | **23.5** |
| B | **40.8** |
| C | **39.7** |
| Comparative Formula with 0.3% triclosan | **41.0** |

The formula containing stannous fluoride, zinc oxide/zinc citrate and 0.1% Zingerone (Formula B) showed a high % reduction of PGE2 (40.8%). A comparative toothpaste formulation containing triclosan inhibited PGE2 to approximately the same extent (41%). The formulation containing stannous fluoride, zinc oxide and zinc citrate showed a 23.5% reduction of inflammation. These data show that the addition of 0.1% of Zingerone boosts the anti-inflammatory effect of the stannous and zinc ions to a great extent, which is at parity to formulation containing triclosan.

### Example 3

### Determination of anti-bacterial efficacy of toothpaste formulas

Antibacterial efficacy of several formulations were determined in an oral biofilm model. The formulations tested included: a) 1% zinc oxide, 0.5% zinc citrate trihydrate, and 0.454% stannous fluoride (which contains 0.344% stannous ions) with 0.1% zingerone; b) 0.5 zinc citrate trihydrate, and 0.454% stannous fluoride without zingerone; c) a formulation containing 0.454% stannous fluoride, and lacking zinc and d) a commercial formulation containing zinc lactate and 0.454% stannous fluoride.

Oral biofilm growth was initiated by incubating 24 HAP (hydroxyapatite) disks attached to a 24 well plate lid in 5% stimulated salivary inoculum in McBain media at 37°C in 5% CO₂. The biofilm was grown for 48 hours and fresh media was replaced after 24 hours. The 48 hour biofilms were treated with 1:3 toothpaste slurries for 2 minutes, and then rinsed by dipping the plate twice for 10 seconds in DI water. All treated biofilms were incubated in 0.03% TSB, 0.5% sucrose at pH 7.2. The pH change was measured for each biofilm after 6 hours of incubation in the above media.

The data indicated that the formulations containing zinc oxide/zinc citrate/stannous ions, and zinc oxide/zinc citrate/stannous ions/zingerone both provided antibacterial activity at parity with the commercial triclosan-containing formulation. Thus, the anti-inflammatory effect of zingerone described in Example 2 does not impair the antibacterial activity of the stannous / zinc oxide / zinc citrate combination.

### Example 4

### Uptake of Zn and Sn to Soft Tissue

The uptake of Zn and Sn ions onto soft tissue was measured on three samples of MatTek GIN-606 tissue. The sample formulations included a) a commercial formulation containing zinc lactate and 0.454% stannous fluoride; b) a formulation containing 1.0% zinc oxide, 0.5% zinc citrate, 0.454% stannous fluoride and 1.5% arginine; and c) a formulation containing 1% zinc oxide, 0.5% zinc citrate trihydrate, and 0.454% stannous fluoride with 0.1% zingerone.

After 2 minutes of treatment and three washes, tissues were incubated overnight. Tissues were collected and digested with 0.5ml of mixture of HCl and HNO₃ overnight. Digested samples were diluted to 5 ml by adding distilled water. The samples were then centrifuged and supernatants were submitted for Sn and Zn quantification analysis by using ICP. The samples were compared with an untreated control.

The data for the formulas are shown below:

| Formulas (Sn uptake) | Average (ppm) | Std Dev |
|---|---|---|
| A | 0.0367 | 0.0115 |
| B | 0.0333 | 0.0058 |
| C | 0.02 | 0 |

| Formulas (Zn uptake) | Average (ppm) | Std Dev |
|---|---|---|
| A | 0.42 | 0.026 |
| B | 0.75 | 0.07 |
| C | 0.82 | 0.085 |

The data show that the formulation containing zinc oxide, zinc citrate, stannous ions and zingerone delivered relatively low levels of Sn to soft tissue, and more Zn to soft tissue than the commercial formulation containing zinc lactate and stannous ions.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

## Claims

1. An oral care composition comprising:
a. a phenolic alkenone comprising zingerone, where the zingerone is present in an amount from 0.01 to 1 wt %, based on the total weight of the composition,
b. zinc oxide;
c. zinc citrate; and
d. a stannous ion source, wherein the source of stannous ions is stannous fluoride in an amount of 0.1 to 2 wt %, based on the total weight of the composition;
wherein the zinc citrate is present in an amount of from 0.25 to 1.0 wt % and zinc oxide is present in an amount of from 0.75 to 1.25 wt %, based on the total weight of the composition.

2. The oral care composition of any of the preceding claims, wherein the zinc citrate is present in an amount of about 0.5 wt % and zinc is present in an amount of about 1.0% based on the total weight of the composition.

3. The oral care composition of any of the preceding claims further comprising synthetic amorphous precipitated abrasive silica in an amount of from 1 to 25 wt %, based on the total weight of the composition.

4. The oral care composition of any of the preceding claims further comprising a high cleaning silica in an amount of from 1 to 15 wt %, based on the total weight of the composition.

5. The oral care composition of any of the preceding claims further comprising an effective amount of one or more alkali phosphate salts, for example sodium tripolyphosphate in an amount of from 1 to 5 wt %, based on the total weight of the composition.

6. The oral care composition of any of the preceding claims further comprising citric acid in an amount of from 0.1 to 3 wt %, and citrate ion, for example trisodium citrate dihydrate, in an amount of from 0.1 to 5 wt %, based on the total weight of the composition.

7. The oral care composition of any of the preceding claims further comprising carboxymethyl cellulose in an amount of from 0.1 to 1.5 wt %, based on the total weight of the composition.

8. The oral care composition of any of the preceding claims further comprising an anionic surfactant, e.g., sodium lauryl sulfate, in an amount of from 0.5 to 5 wt %, based on the total weight of the composition, and optionally an amphoteric surfactant in an amount of from 0.5 to 5 wt %, based on the total weight of the composition.

9. The oral care composition of any of the preceding claims, further comprising a PVM/MA copolymer, in an amount of from 0.1 to 5 wt %, based on the total weight of the composition.

10. The oral care composition of any of the preceding claims, further comprising from 5 to 40 wt % free water, based on the total weight of the composition.

11. The oral care composition of any of the preceding claims, comprising:
a. about 0.1 to 0.3 wt % zingerone;
b. about 1.0 wt % zinc oxide;
c. about 0.5 wt % zinc citrate; and
d. about 0.4 to 0.5 wt % stannous fluoride.

12. The oral care composition of any of the preceding claims, comprising:
e. about 0.1 to 0.3 wt % zingerone;
f. about 1.0 wt % zinc oxide;
g. about 0.5 wt % zinc citrate;
h. about 0.4 to 0.5 wt % stannous fluoride; and
i. about 12 wt % abrasive silica.

13. The oral care composition of any of the preceding claims, wherein the oral composition may be any of the following oral compositions selected from the group consisting of: a toothpaste or a dentifrice, a mouthwash or a mouth rinse, a topical oral gel, a chewing gum, and a denture cleanser.

14. The oral care composition of any of the preceding claims, wherein the composition is obtained or obtainable by combining the ingredients as set forth in any of the preceding compositions.

15. The oral composition of any of the preceding claims for use in a method to improve oral health comprising applying an effective amount of the composition to the oral cavity, wherein the method is effective to:
i. reduce or inhibit formation of dental caries, and/or
ii. clean the teeth and oral cavity.

## Patentansprüche

1. Mundpflegezusammensetzung, umfassend:
a. ein phenolisches Alkenon, umfassend Zingeron, wobei das Zingeron in einer Menge von 0,01 bis 1 Gew.-%, bezogen auf das Gesamtgewicht der Zusammensetzung, vorliegt,
b. Zinkoxid;
c. Zinkcitrat; und
d. eine Zinnionenquelle, wobei die Zinnionenquelle Zinnfluorid in einer Menge von 0,1 bis 2 Gew.-%, bezogen auf das Gesamtgewicht der Zusammensetzung, ist;
wobei das Zinkcitrat in einer Menge von 0,25 bis 1,0 Gew.-% und Zinkoxid in einer Menge von 0,75 bis 1,25 Gew.-%, bezogen auf das Gesamtgewicht der Zusammensetzung, vorliegt.

2. Mundpflegezusammensetzung nach einem beliebigen der vorhergehenden Ansprüche, wobei das Zinkcitrat in einer Menge von etwa 0,5 Gew.-% und Zink in einer Menge von etwa 1,0 %, bezogen auf das Gesamtgewicht der Zusammensetzung, vorliegt.

3. Mundpflegezusammensetzung nach einem beliebigen der vorhergehenden Ansprüche, weiterhin umfassend synthetisches, amorphes, gefälltes, abrasives Silica in einer Menge von 1 bis 25 Gew.-%, bezogen auf das Gesamtgewicht der Zusammensetzung.

4. Mundpflegezusammensetzung nach einem beliebigen der vorhergehenden Ansprüche, weiterhin umfassend ein stark reinigendes Silica in einer Menge von 1 bis 15 Gew.-%, bezogen auf das Gesamtgewicht der Zusammensetzung.

5. Mundpflegezusammensetzung nach einem beliebigen der vorhergehenden Ansprüche, weiterhin umfassend eine wirksame Menge eines oder mehrerer Alkaliphosphatsalze, zum Beispiel Natriumtripolyphosphat in einer Menge von 1 bis 5 Gew.-%, bezogen auf das Gesamtgewicht der Zusammensetzung.

6. Mundpflegezusammensetzung nach einem beliebigen der vorhergehenden Ansprüche, weiterhin umfassend Zitronensäure in einer Menge von 0,1 bis 3 Gew.-% und Citrat-Ionen, zum Beispiel Trinatriumcitrat-Dihydrat, in einer Menge von 0,1 bis 5 Gew.-%, bezogen auf das Gesamtgewicht der Zusammensetzung.

7. Mundpflegezusammensetzung nach einem beliebigen der vorhergehenden Ansprüche, weiterhin umfassend Carboxymethylcellulose in einer Menge von 0,1 bis 1,5 Gew.-%, bezogen auf das Gesamtgewicht der Zusammensetzung.

8. Mundpflegezusammensetzung nach einem beliebigen der vorhergehenden Ansprüche, weiterhin umfassend ein anionisches oberflächenaktives Mittel, z.B. Natriumlaurylsulfat, in einer Menge von 0,5 bis 5 Gew.-%, bezogen auf das Gesamtgewicht der Zusammensetzung, und gegebenenfalls ein amphoteres oberflächenaktives Mittel in einer Menge von 0,5 bis 5 Gew.-%, bezogen auf das Gesamtgewicht der Zusammensetzung.

9. Mundpflegezusammensetzung nach einem beliebigen der vorhergehenden Ansprüche, weiterhin umfassend ein PVM/MA-Copolymer in einer Menge von 0,1 bis 5 Gew.-%, bezogen auf das Gesamtgewicht der Zusammensetzung.

10. Mundpflegezusammensetzung nach einem beliebigen der vorhergehenden Ansprüche, weiterhin umfassend von 5 bis 40 Gew.-% freies Wasser, bezogen auf das Gesamtgewicht der Zusammensetzung.

11. Mundpflegezusammensetzung nach einem beliebigen der vorhergehenden Ansprüche, umfassend:
a. etwa 0,1 bis 0,3 Gew.-% Zingeron;
b. etwa 1,0 Gew.-% Zinkoxid
c. etwa 0,5 Gew.-% Zinkcitrat; und
d. etwa 0,4 bis 0,5 Gew.-% Zinnfluorid.

12. Mundpflegezusammensetzung nach einem beliebigen der vorhergehenden Ansprüche, umfassend:
e. etwa 0,1 bis 0,3 Gew.-% Zingeron;
f. etwa 1,0 Gew.-% Zinkoxid
g. etwa 0,5 Gew.-% Zinkcitrat;
h. etwa 0,4 bis 0,5 Gew.-% Zinnfluorid; und
i. etwa 12 Gew.-% abrasives Silica.

13. Mundpflegezusammensetzung nach einem beliebigen der vorhergehenden Ansprüche, wobei die Mundpflegezusammensetzung eine beliebige der folgenden oralen Zusammensetzungen ausgewählt aus der Gruppe bestehend aus: einer Zahnpasta oder einem Zahnputzmittel, einem Mundwasser oder einer Mundspülung, einem topischen Mundgel, einem Kaugummi und einem Gebissreiniger sein kann.

14. Mundpflegezusammensetzung nach einem beliebigen der vorhergehenden Ansprüche, wobei die Zusammensetzung durch Kombinieren der Bestandteile, wie sie in einer der vorhergehenden Zusammensetzungen angegeben sind, erhalten wird oder erhältlich ist.

15. Orale Zusammensetzung nach einem beliebigen der vorhergehenden Ansprüche zur Verwendung in einem Verfahren um die Mundgesundheit zu verbessern, umfassend das Auftragen einer wirksamen Menge der Zusammensetzung in die Mundhöhle, wobei das Verfahren wirksam ist, um:
i. die Bildung von Zahnkaries zu reduzieren oder zu verhindern, und/oder
ii. die Zähne und Mundhöhle zu reinigen.

## Revendications

1. Composition de soins bucco-dentaires comprenant :
a. une alcénone phénolique comprenant de la zingérone, où la zingérone est présente en une quantité de 0,01 à 1 % en poids, par rapport au poids total de la composition,
b. oxyde de zinc ;
c. citrate de zinc ; et
d. une source d'ions stanneux, dans laquelle la source d'ions stanneux est du fluorure stanneux en une quantité de 0,1 à 2 % en poids, par rapport au poids total de la composition ;
dans laquelle le citrate de zinc est présent en une quantité de 0,25 à 1,0 % en poids et l'oxyde de zinc est présent en une quantité de 0,75 à 1,25 % en poids, par rapport au poids total de la composition.

2. Composition de soins bucco-dentaires selon l'une quelconque des revendications précédentes, dans laquelle le citrate de zinc est présent en une quantité d'environ 0,5 % en poids et le zinc est présent en une quantité d'environ 1,0 % par rapport au poids total de la composition.

3. Composition de soins bucco-dentaires selon l'une quelconque des revendications précédentes, comprenant en outre de la silice abrasive précipitée amorphe synthétique en une quantité de 1 à 25 % en poids, par rapport au poids total de la composition.

4. Composition de soins bucco-dentaires selon l'une quelconque des revendications précédentes, comprenant en outre une silice hautement nettoyante en une quantité de 1 à 15 % en poids, par rapport au poids total de la composition.

5. Composition de soins bucco-dentaires selon l'une quelconque des revendications précédentes, comprenant en outre une quantité efficace d'un ou plusieurs sels de phosphate alcalin, par exemple le tripolyphosphate de sodium en une quantité de 1 à 5 % en poids, par rapport au poids total de la composition.

6. Composition de soins bucco-dentaires selon l'une quelconque des revendications précédentes, comprenant en outre de l'acide citrique en une quantité de 0,1 à 3 % en poids, et un ion citrate, par exemple du citrate trisodique dihydraté, en une quantité de 0,1 à 5 % en poids, par rapport au poids total de la composition.

7. Composition de soins bucco-dentaires selon l'une quelconque des revendications précédentes, comprenant en outre de la carboxyméthylcellulose en une quantité de 0,1 à 1,5 % en poids, par rapport au poids total de la composition.

8. Composition de soins bucco-dentaires selon l'une quelconque des revendications précédentes, comprenant en outre un tensioactif anionique, par exemple du laurylsulfate de sodium, en une quantité de 0,5 à 5 % en poids, par rapport au poids total de la composition, et éventuellement un tensioactif amphotère en une quantité de 0,5 à 5 % en poids, par rapport au poids total de la composition.

9. Composition de soins bucco-dentaires selon l'une quelconque des revendications précédentes, comprenant en outre un copolymère PVM/MA, en une quantité de 0,1 à 5 % en poids, par rapport au poids total de la composition.

10. Composition de soins bucco-dentaires selon l'une quelconque des revendications précédentes, comprenant en outre de 5 à 40 % en poids d'eau libre, par rapport au poids total de la composition.

11. Composition de soins bucco-dentaires selon l'une quelconque des revendications précédentes, comprenant :
a. environ 0,1 à 0,3 % en poids de zingérone ;
b. environ 1,0 % en poids d'oxyde de zinc ;
c. environ 0,5 % en poids de citrate de zinc ; et
d. environ 0,4 à 0,5 % en poids de fluorure stanneux.

12. Composition de soins bucco-dentaires selon l'une quelconque des revendications précédentes, comprenant :
e. environ 0,1 à 0,3 % en poids de zingérone ;
f. environ 1,0 % en poids d'oxyde de zinc ;
g. environ 0,5 % en poids de citrate de zinc ;
h. environ 0,4 à 0,5 % en poids de fluorure stanneux ; et
i. environ 12 % en poids de silice abrasive.

13. Composition de soin buccal selon l'une quelconque des revendications précédentes, dans laquelle la composition orale peut être l'une quelconque des compositions orales suivantes choisie dans le groupe constitué par : une pâte dentifrice ou un dentifrice, un bain de bouche ou un rince-bouche, un gel oral topique, une gomme à mâcher et un nettoyant pour prothèses dentaires.

14. Composition de soins bucco-dentaires selon l'une quelconque des revendications précédentes, dans laquelle la composition est obtenue ou peut être obtenue en combinant les ingrédients tels qu'indiqués dans l'une quelconque des compositions précédentes.

15. Composition orale selon l'une quelconque des revendications précédentes pour une utilisation dans un procédé pour améliorer la santé bucco-dentaire comprenant l'application d'une quantité efficace de la composition à la cavité buccale, dans laquelle le procédé est efficace pour :
i. réduire ou inhiber la formation de caries dentaires, et/ou
ii. nettoyer les dents et la cavité buccale.
